Europäisches Patentamt

European Patent Office

Office européen des brevets

(19)

(11) Numéro de publication : **0 086 706**
**B1**

(12) # FASCICULE DE BREVET EUROPÉEN

(45) Date de publication du fascicule du brevet :
07.08.85

(51) Int. Cl.⁴ : **C 12 M   1/16**

(21) Numéro de dépôt : 83400267.7

(22) Date de dépôt : 08.02.83

(54) **Trepan semi-automatique pour réaliser des cavités dans une couche de matière gélifiée.**

(30) Priorité : 09.02.82 FR 8202057

(43) Date de publication de la demande :
24.08.83 Bulletin 83/34

(45) Mention de la délivrance du brevet :
07.08.85 Bulletin 85/32

(84) Etats contractants désignés :
AT BE CH DE FR GB IT LI LU NL SE

(56) Documents cités :
FR-A- 2 321 902
GB-A- 1 361 401
GB-A- 2 067 126
US-A- 2 463 455
US-A- 3 971 098

(73) Titulaire : **RHONE-POULENC S.A.**
**25, quai Paul Doumer**
**F-92408 Courbevoie (FR)**

(72) Inventeur : **Lissot, Jean**
**5, Place du Val Clos**
**F-77170 Brie-Comte-Robert (FR)**
Inventeur : **Pascal, Claude**
**24, rue de Moissy**
**F-77380 Combs La Ville (FR)**

(74) Mandataire : **Pilard, Jacques et al**
**RHONE-POULENC RECHERCHES Service Brevets**
**Pharma 25, Quai Paul Doumer**
**F-92408 Courbevoie Cedex (FR)**

## Description

La présente invention concerne un dispositif semi-automatique permettant de réaliser des cavités cylindriques selon une disposition géométrique donnée dans une couche de matière gélifiée de consistance variable et de faible cohésion, en vue de la détermination de dosages microbiologiques par diffusion.

Un antibiotique possède une activité inhibitrice vis-à-vis des microorganismes vivants et cette propriété peut être mise à profit pour sa détection et son dosage au cours des différentes phases de sa fabrication.

Parmi les méthodes classiquement utilisées pour les dosages microbiologiques est la méthode de diffusion qui consiste à placer à la surface de plaques de milieu gélosé et ensemencé, en général en boîtes de Pétri, des solutions de concentrations progressives de l'antibiotique de raison donnée, généralement 1,5 ou 2. Après incubation, il apparaît des zones circulaires d'inhibition dont le diamètre est en relation linéaire avec le logarithme des concentrations. Le dosage est réalisé par comparaison des réponses obtenues pour l'échantillon avec celles d'un étalon.

En raison du caractère aléatoire des réponses biologiques, un dosage microbiologique doit faire l'objet de répétitions. Pour tenir compte de certains facteurs de variation non maîtrisables, il est nécessaire d'adopter des plans expérimentaux permettant de les prendre en considération. Ainsi, pour effectuer un seul dosage, il est habituel d'utiliser 10 à 20 boîtes de Pétri correspondant à 30 à 60 réponses pour l'étalon. De plus, pour les mêmes raisons, un dosage doit être répété plusieurs jours de suite pour obtenir la précision désirée.

Il est particulièrement important de pouvoir automatiser le plus possible la méthode étant donné le grand nombre de déterminations qui doivent être enregistrées. Par ailleurs, l'automatisation permet de diminuer de façon notable les risques d'erreurs.

Pour la mise en œuvre de la méthode de diffusion, il est nécessaire de réaliser au sein du milieu nutritif ensemencé des cavités cylindriques, destinés à recevoir les solutions d'antibiotique, qui doivent être identiques, présenter des parois lisses et ne pas contenir de particules de matières résiduelles. De plus, lors du découpage de ces cavités, il est particulièrement important de ne pas décoller la matière gélifiée, telle que la gélose, du fond du récipient qui la contient. De cette manière, il est possible d'obtenir une diffusion uniforme de l'antibiotique à travers la matière gélifiée, ce qui permet une limitation nette de la zone d'inhibition.

Le brevet anglais GB 1 361 401 concerne un dispositif destiné à réaliser des cavités dans un gel constitué d'un tube dont l'extrémité inférieure est coupante et à l'intérieur duquel est fixé un second tube dans lequel est créée une dépression et dont l'extrémité inférieure est suffisamment proche de la surface du gel pour que la matière puisse être aspirée. L'utilisation d'un tel dispositif nécessite des essais préalables de réglage de la hauteur du tube intérieur qui est fonction de l'épaisseur de la matière à percer.

Le brevet américain US 2 463 455 concerne un dispositif reposant sur un principe analogue dans lequel le tube intérieur est réglé de telle façon que son extrémité inférieure pénètre tout juste la surface du gel.

Il a été trouvé, et c'est ce qui fait l'objet de la présente invention, que des cavités présentant les caractéristiques requises peuvent être réalisées simultanément au moyen d'un dispositif semi-automatique décrit plus complètement ci-après qui permet en outre une distribution symétrique de ces cavités à la surface de la matière gélifiée.

Le dispositif selon l'invention est constitué par l'assemblage de plusieurs dispositifs élémentaires dont chacun se compose :

a) d'un emporte-pièce dont le diamètre extérieur est généralement compris entre 2 et 12 mm et dont l'extrémité inférieure, qui constitue le couteau emporte-pièce, est biseautée vers l'intérieur du cylindre de façon à avoir un bord de coupe très effilé, et

b) d'un tube d'aspiration dont le diamètre extérieur est légèrement inférieur au diamètre intérieur de l'emporte-pièce, qui coulisse dans l'emporte-pièce et qui est relié à un système permettant de créer une dépression dans ce tube par l'intermédiaire d'un récipient destiné à recueillir la matière gélifiée éliminée, l'extrémité inférieure du tube d'aspiration étant crénelée.

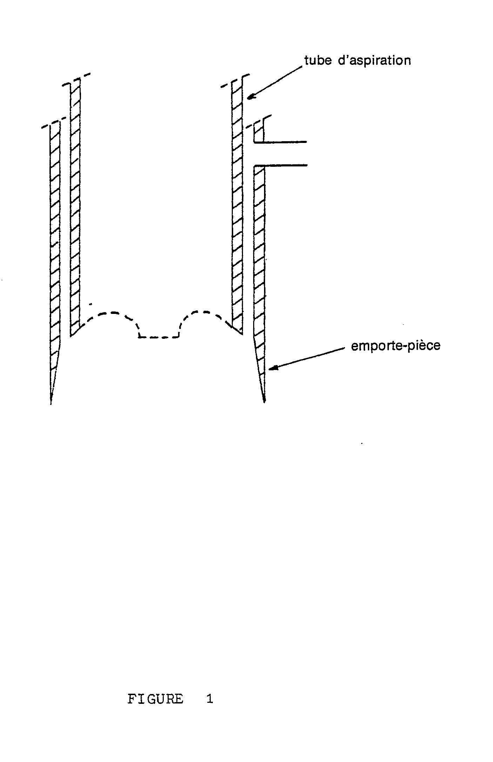

La figure 1 donne le schéma d'un dispositif élémentaire.

Selon l'invention, la réalisation d'une cavité par un dispositif élémentaire s'effectue en quatre phases successives qui sont les suivantes :

a) l'emporte-pièce pénètre perpendiculairement à la surface de la gélose jusqu'à entrer en contact avec le fond du récipient contenant la matière gélifiée.

b) le tube d'aspiration, dans lequel est créée une dépression, descend au contact de la matière gélifiée puis pénètre jusqu'à entrer en contact avec le fond du récipient. Compte tenu de la dépression créée, la matière gélifiée se trouve aspirée dans le tube au fur et à mesure de sa descente. Les crénelures de la partie inférieure du tube d'aspiration permettent à l'air qui se trouve, sous la pression atmosphérique, entre les deux tubes coulissants, de passer dans le tube d'aspiration lorsque celui-ci est en contact avec le fond du récipient.

c) le tube d'aspiration remonte puis la pression atmosphérique se rétablit à l'intérieur du tube d'aspiration, puis

d) l'emporte-pièce remonte à son tour dégageant ainsi la cavité.

Pour que le dispositif fonctionne dans les meilleures conditions afin d'obtenir des cavités de qualité, l'emporte-pièce, qui joue le rôle de couteau, doit répondre à certaines exigences. En particulier, lors de la pénétration dans la couche de gélose, il doit trancher sans comprimer ni déchirer ce qui implique un bord de coupe très effilé et un parfait état de sa paroi extérieure. De cette manière, il est possible d'obtenir une cavité dont la paroi est lisse. De plus, l'état de la surface extérieure de l'emporte-pièce doit être tel que l'adhérence de l'emporte-pièce à la matière gélifiée soit minimale afin d'éviter le décollement de la matière gélifiée du fond du récipient lors du retrait de l'emporte-pièce. Enfin, l'emporte-pièce doit venir en contact avec le fond du récipient et être aussi jointif que possible afin d'éviter la détérioration du fond de la cavité lors de l'aspiration de la matière gélifiée.

Le tube d'aspiration central a pour fonction d'aspirer la matière gélifiée découpée par le couteau pendant que ce dernier est maintenu en place.

Ce tube d'aspiration central coulisse à l'intérieur de l'emporte-pièce. Juste avant l'entrée en contact du tube d'aspiration central avec la matière gélifiée, une dépression est créée à l'intérieur de celui-ci, la pression atmosphérique étant maintenue entre les deux tubes par l'intermédiaire de canaux ménagés dans la paroi de l'emporte-pièce. Du fait de la dépression existant dans le tube d'aspiration, la matière gélifiée est aspirée au fur et à mesure de la descente du tube d'aspiration et elle est entraînée jusqu'au récipient récepteur. Dans ces conditions, toute la pastille de matière gélifiée est aspirée sans laisser de particules solides. Lorsque le tube d'aspiration intérieur remonte et avant que l'emporte-pièce extérieur ne quitte le fond du récipient contenant la matière gélifiée, la pression atmosphérique est rétablie dans le tube d'aspiration.

Selon la présente invention, il est possible de réaliser simultanément des cavités identiques réparties symétriquement par rapport au centre du récipient contenant la matière gélifiée.

Les tubes d'aspiration intérieurs sont rendus solidaires par l'intermédiaire d'une plaque support montée sur un arbre et coulissant verticalement le long de cet arbre.

Les emporte-pièces extérieurs sont supportés par une autre plaque permettant le coulissement de l'ensemble des emporte-pièces extérieurs sur l'ensemble des tubes d'aspiration intérieurs. Les emporte-pièces extérieurs sont maintenus vers le bas au moyen de ressorts.

Les cavités étant réalisées simultanément, les tubes d'aspiration intérieurs sont raccordés en parallèle au récipient collecteur de matière gélifiée aspirée. Les tuyaux souples de raccordement des tubes d'aspiration intérieurs au récepteur de matière gélifiée ne sont pas simplement des canalisations mais ils remplissent une fonction nécessaire pour limiter le débit de l'air aspiré par les tubes d'aspiration intérieurs lorsque toute la matière gélifiée a été emportée, afin d'éviter la création d'une dépression excessive au fond de la cavité et la cassure de la dépression régnant à l'intérieur du récipient récepteur commun alors qu'il ne reste de la matière gélifiée que dans un ou quelques uns seulement des tuyaux de raccordements.

L'ensemble du dispositif permet d'obtenir, à l'aide d'un système d'animation unique, la descente des emporte-pièces et leur pénétration dans la matière gélifiée jusqu'au fond du récipient, la descente des tubes d'aspiration intérieurs et leur remontée ainsi que la remontée des couteaux.

L'animation verticale de l'ensemble est réalisée, par exemple, au moyen d'un système bielle-manivelle entraîné par un moteur électrique à courant continu qui permet d'obtenir des vitesses variables. Le mouvement sinusoïdal obtenu présente l'avantage d'une descente et d'une approche rapide suivies d'un ralentissement pendant les opérations de découpage et d'aspiration de la matière gélifiée. De cette manière la durée totale du cycle peut être réduite de façon appréciable.

Généralement la séquence pression atmosphérique-dépression-pression atmosphérique pendant la descente et la remontée du tube d'aspiration intérieur est réalisée au moyen de deux électro-vannes.

Une opération de découpage est réalisée en un tour du système bielle-manivelle ce qui correspond à une descente et une remontée des emporte-pièces extérieurs. La synchronisation des mouvements est rapportée à la position angulaire du manneton et par conséquent à la cote tube d'aspiration intérieur-récipient par l'intermédiaire d'un programmateur à came couplé à la rotation du moteur.

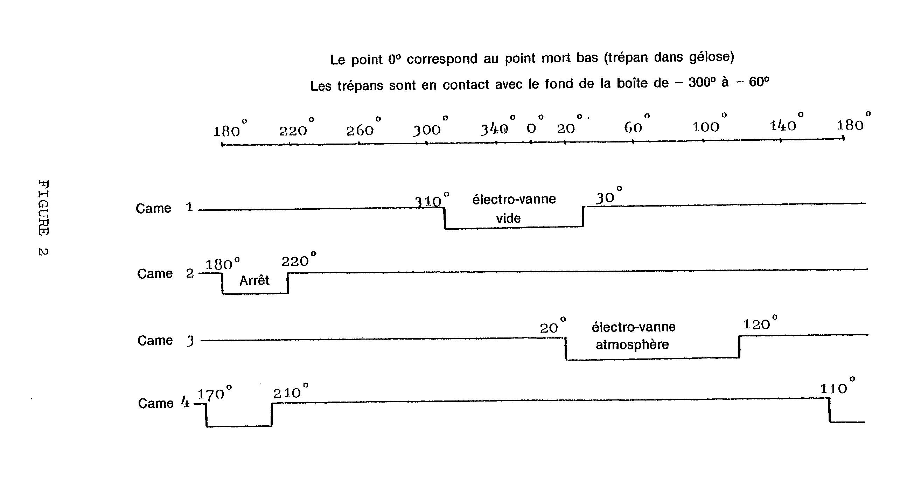

La figure 2 représente le schéma de fonctionnement d'un dispositif élémentaire.

Afin de repérer la position des différentes cavités les unes par rapport aux autres, il est particulièrement avantageux de compléter le dispositif par un système permettant de réaliser une empreinte sur le rebord du récipient contenant la matière gélifiée. Généralement le système est constitué d'un stylet chauffant qui, par application sur le rebord d'un récipient thermoplastique, réalise une encoche qui peut être en forme de V.

Ce dispositif complémentaire fonctionne en synchronisme avec le moyen d'animation de l'ensemble emporte-pièce-tube d'aspiration.

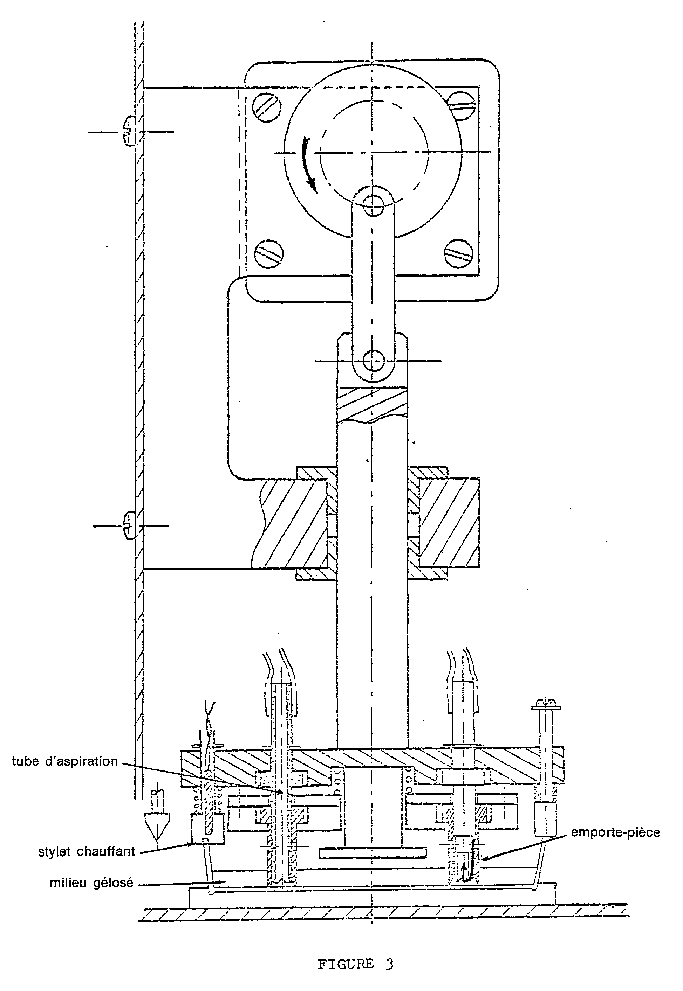

La figure 3 représente l'ensemble du dispositif.

Par ailleurs, lorsque l'on utilise comme récipient de matière gélifiée pour effectuer les dosages une boîte de Pétri, un système à tiroir manuel permet sa mise en place. Lorsque le tiroir est tiré, il autorise l'échange des boîtes sur un support de positionnement. Lorsque le tiroir est repoussé, un verrouillage de fin de course positionne exactement la boîte relativement à l'ensemble du dispositif de découpage et d'aspiration et enclenche automatiquement les différentes phases de l'opération.

**Revendications**

1. Dispositif semi-automatique permettant de réaliser des cavités cylindriques selon une position géométrique donnée dans une couche de matière gélifiée de consistance variable et d'une faible cohésion caractérisé en ce qu'il est constitué par l'assemblage de plusieurs dispositifs élémentaires dont chacun se compose :

a) d'un emporte-pièce dont l'extrémité inférieure, qui constitue le couteau, est biseautée vers l'intérieur du cylindre de façon à avoir un bord de coupe très effilé, et

b) d'un tube d'aspiration, coulissant à l'intérieur de l'emporte-pièce dont l'extrémité inférieure est crénelée qui est relié à un système permettant de créer une dépression à l'intérieur du tube par l'intermédiaire d'un récipient destiné à recueillir la matière gélifiée éliminée, et qui permet la réalisation des quatre phases successives suivantes :

a) pénétration de l'emporte-pièce extérieur perpendiculairement à la surface de la matière gélifiée jusqu'à entrer en contact avec le fond du récipient contenant la matière gélifiée

b) descente du tube d'aspiration dans lequel est créée une dépression au contact de la matière gélifiée et pénétration jusqu'à entrer en contact avec le fond du récipient

c) remontée du tube d'aspiration intérieur et rétablissement de la pression atmosphérique, et

d) remontée de l'emporte-pièce extérieur.

2. Dispositif selon la revendication 1, caractérisé en ce que les tubes d'aspiration intérieurs sont rendus solidaires par l'intermédiaire d'un support monté sur un arbre et coulissant le long de cet arbre, et les emporte-pièces extérieurs sont supportés par un autre support permettant le coulissement de l'ensemble des emporte-pièces extérieurs sur l'ensemble des tubes d'aspiration intérieurs.

3. Dispositif selon la revendication 1, caractérisé en ce qu'il est animé par un système bielle-manivelle entraîné par un moteur électrique à courant continu et que la séquence pression atmosphérique-dépression-pression atmosphérique est réalisée au moyen de deux électro-vannes.

4. Dispositif selon la revendication 1, caractérisé en ce qu'il comporte un système constitué d'un stylet chauffant qui réalise un repère sur le rebord du récipient contenant la matière gélosée afin de reconnaître les positions relatives des cavités.

## Claims

1. A semi-automatic device which makes it possible to produce cylindrical cavities, according to a given geometrical arrangement, in a layer of gelled material of variable consistency and weak cohesion, characterised in that it consists of a set of several individual devices each composed of :

a) a cutter of which the lower end, which forms the blade, is bevelled towards the inside of the cylinder so as to give a very sharp cutting edge, and

b) a suction tube which slides inside the cutter, the lower end of which is notched and which is connected to a system making it possible to create a pressure reduction inside the tube, via a container for collecting the gelled material removed,

and which makes it possible to carry out the following four successive stages :

a) causing the outer cutter to enter perpendicularly to the surface of the gelled material until it comes into contact with the bottom of the vessel containing the gelled material,

b) lowering the suction tube in which a pressure reduction is created when it comes into contact with the gelled material, and causing it to enter until it comes into contact with the bottom of the container,

c) raising the inner suction tube and reestablishing atmospheric pressure, and

d) raising the outer cutter.

2. A device according to Claim 1, characterised in that the inner suction tubes are fixed together by means of a support fitted to a shaft and sliding along this shaft, and the outer cutters are supported by another support enabling the assembly of outer cutters to slide over the assembly of inner suction tubes.

3. A device according to Claim 1, characterised in that it is driven by a crank-connecting rod system driven by a direct-current electric motor, and the sequence atmospheric pressure-reduced pressure-atmospheric pressure is achieved by means of two electrovalves.

4. A device according to Claim 1, characterised in that it includes a system consisting of a heated probe which produces a mark on the rim of the vessel containing the agar material, so as to identify the relative positions of the cavities.

## Patentansprüche

1. Halbautomatische Vorrichtung, die es ermöglicht, zylindrische Hohlräume entsprechend einer gegebenen geometrischen Anordnung in einer Schicht aus Gelmaterial mit variabler Konsistenz und geringer Kohäsion herzustellen, dadurch gekennzeichnet, daß sie aus einem Satz mehrerer Einzelvorrichtungen besteht, von denen jede umfaßt :

a) einen Lochstanzer, dessen unteres Ende, das die Schneide bildet, an der Innenseite des Zylinders derart zugeschliffen ist, daß eine sehr scharfe Schneidkante entsteht, und

b) ein Saugrohr, das im Inneren des Lochstanzers gleitet und dessen unterer Rand ausgezackt ist und das an ein System angeschlossen ist, das die Erzeugung eines Unterdrucks im Inneren des Rohrs unter Zwischenschaltung eines Behälters zur Aufnahme des entfernten Gelmaterials ermöglicht,

und die Durchführung der folgenden aufeinanderfolgenden Phasen ermöglicht :

a) Eindringen des äußeren Lochstanzers senkrecht zur Oberfläche des Gelmaterials bis Kontakt mit dem Boden des das Gelmaterial enthaltenden Gefäßes erzielt ist,

b) Absenken des Saugrohres, in dem ein Unterdruck bei Kontakt mit dem Gelmaterial erzeugt wird, und Eindringen, bis Kontakt mit dem Boden des Gefäßes erzielt ist,

c) Heben des inneren Saugrohres und Wiederherstellung von Atmosphärendruck, und

d) Heben des äußeren Lochstanzers.

2. Vorrichtung nach Anspruch 1, dadurch gekennzeichnet, daß die inneren Saugrohre an einer gemeinsamen Trägerplatte angebracht sind, die an einer Stange befestigt ist und vertikal entlang dieser Stange gleitet, und daß die äußeren Lochstanzer an einer anderen Platte befestigt sind, die das Gleiten sämtlicher äußeren Lochstanzer entlang sämtlicher inneren Saugrohre ermöglicht.

3. Vorrichtung nach Anspruch 1, dadurch gekennzeichnet, daß sie durch ein Kurbelstangensystem bewegt wird, das von einem kontinuierlich laufenden Elektromotor angetrieben ist, und daß die Folge Atmosphärendruck/Unterdruck/Atmosphärendruck mittels zweier Elektroventile verwirklicht wird.

4. Vorrichtung nach Anspruch 1, dadurch gekennzeichnet, daß sie ein System, bestehend aus einem Heizstab, der eine Kerbe am Rand des das Gelmaterial enthaltenden Gefäßes erzeugt, um die Relativlagen der Hohlräume wiederzufinden, trägt.

tube d'aspiration

emporte-pièce

FIGURE 1

Le point 0° correspond au point mort bas (trépan dans gélose)

Les trépans sont en contact avec le fond de la boîte de − 300° à − 60°

$180°$   $220°$   $260°$   $300°$   $340°$  $0°$  $20°$   $60°$   $100°$   $140°$   $180°$

Came 1 ——————————————————— $310°$  électro-vanne  $30°$ ——————————
                                              vide

Came 2 —$180°$  $220°$ ——————————————————————————————————
            Arrêt

Came 3 ——————————————————————— $20°$  électro-vanne  $120°$ ————————
                                              atmosphère

Came 4 —$170°$  $210°$ ——————————————————————— $110°$ ———

FIGURE 2

2

0 086 706

tube d'aspiration

stylet chauffant

milieu gélosé

emporte-pièce

FIGURE 3